(19) **Europäisches Patentamt / European Patent Office / Office européen des brevets**

(11) **EP 4 335 930 A1**

(12) # EUROPEAN PATENT APPLICATION

(43) Date of publication:
**13.03.2024 Bulletin 2024/11**

(21) Application number: **22217192.8**

(22) Date of filing: **29.12.2022**

(51) International Patent Classification (IPC):
**C12Q 1/6886** (2018.01)     **C12Q 1/6851** (2018.01)

(52) Cooperative Patent Classification (CPC):
(C-Sets available)
**C12Q 1/6886; C12Q 1/6851;** C12Q 2600/16

(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.09.2022 CN 202211081696**

(71) Applicant: **Fuzhou Maixin Biotech Co., Ltd. China 350108 Fuzhou Fujian (CN)**

(72) Inventors:
- **MENG, Chun**
  **Fuzhou, 350108 (CN)**
- **HONG, Jing**
  **Fuzhou, 350108 (CN)**
- **GUO, Liang**
  **Fuzhou, 350108 (CN)**
- **MA, Wenxiao**
  **Fuzhou, 350108 (CN)**
- **HUANG, Yiwei**
  **Fuzhou, 350108 (CN)**
- **LIN, Xiaodie**
  **Fuzhou, 350108 (CN)**
- **XIE, Liling**
  **Fuzhou, 350108 (CN)**
- **WANG, Xiaoya**
  **Fuzhou, 350108 (CN)**
- **LIN, Qixin**
  **Fuzhou, 350108 (CN)**

(74) Representative: **Chung, Hoi Kan**
**Mandarin IP Limited**
**7 Cherry Trees**
**Great Shelford**
**Cambridge CB22 5XA (GB)**

Remarks:
The complete document including Reference Table(s) and the Sequence Listing(s) can be downloaded from the EPO website

(54) ## KIT FOR DETECTING MICROSATELLITE INSTABILITY AND METHOD THEREFOR

(57)     The disclosure provides a kit for detecting microsatellite instability and a method therefor. The kit includes a negative control, a plurality of qPCR reaction solutions, a qPCR premix and a sterile enzyme-free water; the plurality of qPCR reaction solutions includes 6 pairs of upstream primers and downstream primers of which the MSI mutation site is amplified, and a reference probe for the internal reference and a detection probe for the mutation site. The difference between the amplification of the gene and the gene at the mutation site of the samples and the negative control is used to detect the microsatellite instability. The method and kit as provided is easy and simple without the need of normal tissues being a control, and the need to open the cap. By doing so, aerosol pollution is avoided and sample supplies are conserved.

Fig. 1

**(Cont. next page)**

(52) Cooperative Patent Classification (CPC): (Cont.)

C-Sets
**C12Q 1/6851, C12Q 2531/113, C12Q 2537/143,
C12Q 2565/101**

**Description**

**FIELD OF THE DISCLOSURE**

The disclosure relates to the technical field of nucleic acid detection (NAT), in particular relates to a kit for detecting microsatellite instability and a method therefor.

**BACKGROUND OF THE DISCLOSURE**

Microsatellites are referred to as short tandem repeats (STRs) having a core sequence consisting of 1-6 bases. These repeats are distributed throughout the human genome. Microsatellite instability (MSI) is the condition of changes in the length of microsatellite repeats that results from the loss of expression of mismatch repair (MMR) genes caused by DNA methylation or gene mutation. It is manifested by changes of the number of repeating units between different individuals, or between normal tissues and certain abnormal tissues of one single individual, which prevents them from playing a normal regulatory role and leads to abnormal cell proliferation and differentiation, then promotes the formation of malignant tumors. A large number of studies have suggested that MSI is closely related to colorectal cancer (CRC), gastric cancer (GC), and endometrial cancer.
In line with the degree of MSI, it can be classified in to three types: high microsatellite instability (MSI-H), low microsatellite instability (MSI-L), and microsatellite stability (MSS). Because MSI is a special type of mutation with multiple additions or deletions of the same bases, methods for detecting MSI are complicated and have great problems in accuracy. At present, MSI detection mostly relies on normal tissue as a control, and there is exposure during the detection, which may easily lead to the contamination of samples by aerosols in the air. Therefore, it is very important and practical to improve the operation convenience, good reference and sensitivity of the detection.

**SUMMARY OF THE DISCLOSURE**

In view of this, the object of the present disclosure is to provide a kit and a method for detecting microsatellite instability with high result accuracy, high detection sensitivity and easy operation.
The following solutions are used for the object of the present disclosure as mentioned above.
A kit for detecting microsatellite instability is provided. The kit includes a negative control, a plurality of qPCR reaction solutions, a qPCR premixes and a sterile enzyme-free water. The plurality of qPCR reaction solutions are applied respectively one on one to detect microsatellite loci NR-21, NR-24, NR-27, MONO-27, BAT-25, and BAT-26.
In some embodiments, the plurality of qPCR reaction solutions includes:

a first qPCR reaction solution for detecting microsatellite loci NR-21; the first qPCR reaction solution has the forward primer shown in SEQ ID No: 1, the reverse primer shown in SEQ ID No: 2, the reference probe shown in SEQ ID No: 3, and the detection probe shown in SEQ ID No: 4; wherein, the nucleotide sequence of SEQ ID No: 3 has a VIC label at its 5' end, and a MGB label at its 3' end; and furthermore, the nucleotide sequence of SEQ ID No: 4 has a FAM label at its 5' end, and has a MGB label at 3' end;

a second qPCR reaction solution for detecting microsatellite loci NR-24; the second qPCR reaction solution has the forward primer shown in SEQ ID No: 5, the reverse primer shown in SEQ ID No: 6, the reference probe shown in SEQ ID No: 7, and the detection probe shown in SEQ ID No: 8; wherein, the nucleotide sequence of SEQ ID No: 7 has a VIC label at its 5' end and a MGB label at its 3' end; and furthermore, the nucleotide sequence of SEQ ID No: 8 has a FAM label at its 5' end, and a MGB label at its 3' end;

a third qPCR reaction solution for detecting microsatellite loci NR-27; the third qPCR reaction solution has the forward primer shown in SEQ ID No: 9, the reverse primer shown in SEQ ID No: 10, the reference probe shown in SEQ ID No: 11, and the detection probe shown in SEQ ID No: 12; wherein, the nucleotide sequence of SEQ ID No: 11 has a VIC label at its 5' end and a MGB label at its 3' end; and furthermore, the nucleotide sequence of SEQ ID No: 12 has a FAM label at its 5' end, and a MGB label at its 3' end;

a fourth qPCR reaction solution for detecting microsatellite loci MONO-27; the fourth qPCR reaction solution has the forward primer shown in SEQ ID No: 13, the reverse primer shown in SEQ ID No: 14, the reference probe shown in SEQ ID No: 15, and the detection probe shown in SEQ ID No: 16; wherein, the nucleotide sequence of SEQ ID No: 15 has a VIC label at its 5' end and a MGB label at its 3' end; and furthermore, the nucleotide sequence of SEQ ID No: 16 has a FAM label at its 5' end, and a MGB label at its 3' end;

a fifth qPCR reaction solution for detecting microsatellite loci BAT-25; the fifth qPCR reaction solution has the forward primer shown in SEQ ID No: 17, the reverse primer shown in SEQ ID No: 18, the reference probe shown in SEQ ID No: 19, and the detection probe shown in SEQ ID No: 20; wherein, the nucleotide sequence of SEQ ID No: 19 has a VIC label at its 5' end and a MGB label at its 3' end; and furthermore, the nucleotide sequence of SEQ ID No:

120 has a FAM label at its 5' end, and a MGB label at its 3' end; and
a sixth qPCR reaction solution for detecting microsatellite loci BAT-26; the sixth qPCR reaction solution has the forward primer shown in SEQ ID No: 21, the reverse primer shown in SEQ ID No: 22, the reference probe shown in SEQ ID No: 23, and the detection probe shown in SEQ ID No: 24; wherein, the nucleotide sequence of SEQ ID No: 23 has a VIC label at its 5' end and a MGB label at its 3' end; and furthermore, the nucleotide sequence of SEQ ID No: 24 has a FAM label at its 5' end, and a MGB label at its 3' end.

In some embodiments, preferably, the negative control is a genomic DNA solution of cell line SW480 at a concentration of 10 ng/μl.

In some embodiments, preferably, the qPCR premixes is Premix Ex Taq, which includes Taq HS, dNTP Mixture, $Mg^{2+}$ and Tli RNaseH.

In some embodiments, preferably, the concentrations of the forward primer, the reverse primer, the reference probe, and the detection probe in the plurality of qPCR reaction solutions are 10 μM.

Based on the above, the present disclosure also provides a non-therapeutic method for detecting microsatellite instability, which includes the above-mentioned kit for detecting microsatellite instability. The method includes the following steps.

S01, taking all components of the kit, mixing the components according to preset conditions, and preparing a plurality of qPCR reaction system mixtures including samples with detection sites, and negative controls;

502, performing qPCR amplification reactions according to preset conditions, and after the reaction, characterizing the plurality of qPCR reaction system mixtures to obtain Ct values having VIC and FAM fluorescence signals; calculating the difference ΔCt for the Ct values of the FAM and VIC signals at the same detection site of the sample;

S03, calculating the difference between the ΔCt value of the sample detection site and the ΔCt value of the negative control at the same site as the sample detection site; namely,

$$\Delta Ct = |Ct_{FAM} - Ct_{VIC}| \quad ;$$

$$\Delta\Delta Ct = |\Delta Ct_{Sample} - \Delta Ct_{Negative}|$$

504, obtaining ΔΔCt and judging it according to preset conditions, and outputting the sample detection result as microsatellite instability MSI or microsatellite stability MSS according to the judgment result.

In some embodiments, preferably, the plurality of qPCR reaction system mixtures are 12 qPCR reaction system mixtures, including 6 samples with detection sites and 6 negative controls, corresponding to the microsatellite loci NR-21, NR-24, NR-27, MONO-27, BAT-25, BAT-26, respectively.

In some embodiments, preferably, the step S01 includes the following steps: taking all components of the kit, melting the components on ice, shaking and mixing them evenly, and then briefly centrifuging for a few seconds to prepare the plurality of qPCR reaction system mixtures including samples with detection sites and negative controls.

In some embodiments, preferably, in the step S02, the conditions for the qPCR amplification action is as follows:

Predenaturation 95° C. for 30 seconds
qPCR reaction 95° C. for 5 seconds, 53 degree annealing extension for 34 seconds, 40 cycles;
wherein, the heating rate is 4° C./s.

In some embodiments, preferably, in the step S04,

with respect to NR-21, when ΔΔCt > 0.15, the site is determined as positive;
with respect to NR-24, when ΔΔCt > 0.25, the site is determined as positive;
with respect to NR-27, when ΔΔCt > 0.54, the site is determined as positive;
with respect to MONO-27, when ΔΔCt > 0.24, the site is determined as positive;
with respect to BAT-25, when ΔΔCt > 0.23, the site is determined as positive;
with respect to BAT-26, when ΔΔCt > 0.51, the site is determined as positive;
when the number of positive sites in the sample is greater than or equal to 2, the sample with the detection sites is determined to be microsatellite instability MSI, otherwise microsatellite stable MSS.

Based on the above, the present disclosure further provides a non-therapeutic method for detecting microsatellite instability in tissues or ctDNA, which includes the above-mentioned method for detecting microsatellite instability.

Based on the above, the present disclosure further provides a non-therapeutic kit for detecting microsatellite instability in tissues or ctDNA, which includes the above-mentioned kit for detecting microsatellite instability.

Compared with the prior art, the present disclosure has the following advantages. The present disclosure provides a kit for detecting microsatellite instability and a method therefor. The kit includes a negative control, a plurality of qPCR reaction solutions, a qPCR premix and a sterile enzyme-free water; the plurality of qPCR reaction solutions includes 6 pairs of upstream primers and downstream primers of which the MSI mutation site is amplified, and a reference probe for the internal reference and a detection probe for the mutation site. The difference between the amplification of the gene and the gene at the mutation site of the samples and the negative control is used to detect the microsatellite instability. The method and kit as provided is easy and simple without the need of normal tissues being a control, and the need to open the cap. By doing so, aerosol pollution is avoided and sample supplies are conserved. The method and kit as disclosed ensures the amplification consistency of the internal reference sites and the detection sites. Additionally, MSI in both tissues and ctDNA, can be detected in the present disclosure with high accuracy and sensitivity.

## BRIEF DESCRIPTION OF DRAWINGS

In order to explain the embodiments of the present invention or the technical solutions in the prior art more clearly, the following briefly introduces the accompanying drawings that need to be used in the description of the embodiments or the prior art. Obviously, the accompanying drawings in the following description are only some embodiments of the present invention. For those of ordinary skill in the art, other drawings can also be obtained according to these drawings without creative efforts.

FIG. 1 is a schematic diagram of the method for detecting microsatellite instability according to the present disclosure.
FIG. 2 is a characterization diagram of the qPCR amplification curve of sample 1 in the embodiment of the present disclosure.
FIG. 3 is Ct values, $\Delta$Ct value, and $\Delta\Delta$Ct values of VIC, FAM fluorescence signals in sample 1 and its corresponding negative control after qPCR amplification reaction.
FIG. 4 is a characterization diagram of the qPCR amplification curve of sample 2 in the embodiment of the present disclosure.
FIG. 5 is Ct values, $\Delta$Ct value, and $\Delta\Delta$Ct values of VIC, FAM fluorescence signals in sample 2 and its corresponding negative control after qPCR amplification reaction.
FIG. 6 is a characterization diagram of the qPCR amplification curve of sample 3 in the embodiment of the present disclosure.
FIG. 7 is Ct values, $\Delta$Ct value, and $\Delta\Delta$Ct values of VIC, FAM fluorescence signals in sample 3 and its corresponding negative control after qPCR amplification reaction.
FIG. 8 is a characterization diagram of the qPCR amplification curve of sample 4 in the embodiment of the present disclosure.
FIG. 9 is Ct values, $\Delta$Ct value, and $\Delta\Delta$Ct values of VIC, FAM fluorescence signals in sample 4 and its corresponding negative control after qPCR amplification reaction.

## DETAILED DESCRIPTION OF THE DISCLOSURE

The present invention will be further described in detail below with reference to the accompanying drawings and embodiments. It is particularly pointed out that the following examples are only used to illustrate the present invention, but do not limit the scope of the present invention. Likewise, the following embodiments are only some rather than all embodiments of the present invention, and all other embodiments obtained by those of ordinary skill in the art without creative work fall within the protection scope of the present invention.

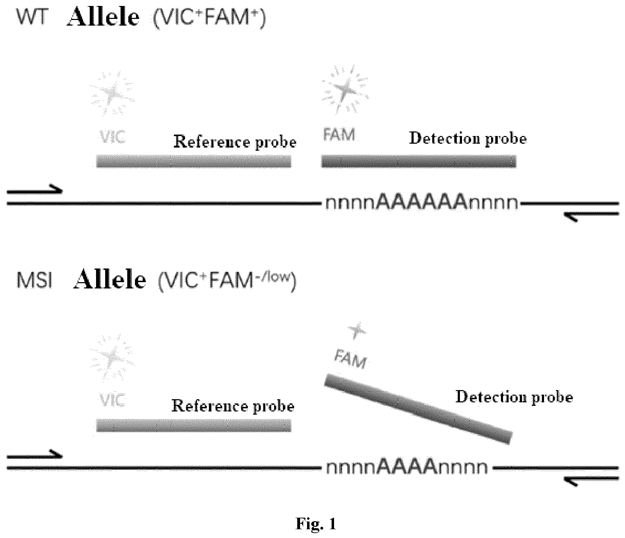

Referred to FIG. 1, a schematic diagram of the method for detecting the microsatellite instability according to the present invention. In the present invention, the microsatellite instability is detected by the amplification difference between the reference probe for amplifying the internal reference gene and the detection probe for amplifying the mutation site. After the amplification reaction, the mixtures of a plurality of qPCR reaction system mixtures were characterized to obtain the Ct values of the VIC and FAM fluorescence signals; then the difference between the $\Delta$Ct value of the sample detection site and the $\Delta$Ct value at the same site of the negative control was calculated. Namely:

$$\Delta Ct = |Ct_{FAM} - Ct_{VIC}| \quad ;$$

$$\Delta\Delta Ct = |\Delta Ct_{Sample} - \Delta Ct_{Negative}|$$

Finally, ΔΔCt is obtained and judged according to preset conditions, and the sample detection result is output as microsatellite instability MSI or microsatellite stability MSS according to the judgment result.

The present invention is further illustrated below in conjunction with specific embodiment 1.

**EMBODIMENT 1**

**Detection of MSI-qPCR of FFPE ctDNA Sample**

(1) Preparation of FFPE ctDNA

Selecting two FFPE samples (sample 1 and sample 2) which are determined as MSI by IHC (immunohistochemical) detection, and two FFPE samples (sample 3 and sample 4) which are determined as MSS by IHC detection.

Cutting, by a microtome, cut 5 to 10 pieces (8 μm each) from the paraffin tissue block, then extracting genomic DNA by FastPure FFPE DNA Isolation Kit (DC105), and quantifying the DNA by Qubit4.0, and diluting the quantified DNA to 10ng/μL to obtain a template for qPCR amplification.

(2) Primers and Probes

The primer and probe sequences for detecting MSI loci in this embodiments is shown in Table 1 below.

Table 1 Primer and Probe Sequences for Detecting MSI Loci

| Name | SEQ ID No . | Sequence(5'-3') | Modification |
|---|---|---|---|
| NR-21-F | SEQ ID No.1 | TTATATTTAAATGTATGTCTC | |
| NR-21-R | SEQ ID No.2 | CAAGCAGATAAAAGAGAAC | |
| NR-21-VIC | SEQ ID No.3 | ACGCGAGTGACCAGAAAGTGT | 5'-VIC, 3'-MGB |
| NR-21-FAM | SEQ ID No.4 | TGGCCTTTTTTTTTTTTTTTTTTTTTTAGCA | 5'-FAM, 3'-MGB |
| NR-24-F | SEQ ID No.5 | GCTGAATTTTACCTCCTGAC | |
| NR-24-R | SEQ ID No.6 | AACCTGGGTGACAGAGTGA | |
| NR-24-VIC | SEQ ID No.7 | AAAAACTCTTCTCTTCCCTG | 5'-VIC, 3'-MGB |
| NR-24-FAM | SEQ ID No.8 | CCTATTTTTTTTTTTTTTTTTTTTTTGTGA | 5'-FAM, 3'-MGB |
| NR-27-F | SEQ ID No.9 | CCAACGTCTGTGAGATCC | |
| NR-27-R | SEQ ID No.10 | ACTAGCAATGACCAATAAGC | |
| NR-27-VIC | SEQ ID No.11 | GGAAACCATGCTTGCAAAC | 5'-VIC, 3'-MGB |
| NR-27-FAM | SEQ ID No.12 | TGGTAAAAAAAAAAAAAAAAAAAAAAAGCCA | 5'-FAM, 3'-MGB |
| MONO-27-F | SEQ ID No.13 | ACTTAATATCCCTAAACAAT | |
| MONO-27-R | SEQ ID No.14 | GCCTGGGTGACATAATGA | |
| MONO-27-VIC | SEQ ID No.15 | ACTACAGGCATGAATTACTACTGT | 5'-VIC, 3'-MGB |
| MONO-27-FAM | SEQ ID No.16 | CTCCAGTTTTTTTTTTTTTTTTTTTTTTTTTTTGA | 5'-FAM, 3'-MGB |
| BAT-25-F | SEQ ID No.17 | CCAAGAATGTAAGTGGGAGTG | |
| BAT-25-R | SEQ ID No.18 | CATTCTGCATTTTAACTATGGCTC | |
| BAT-25-VIC | SEQ ID No.19 | TTCTCTAAAGAGTTTTGTGTTTTGTT | 5'-VIC, 3'-MGB |
| BAT-25-FAM | SEQ ID No.20 | TTGATTTTTTTTTTTTTTTTTTTTTTTTTTGAGAACA | 5'-FAM, 3'-MGB |
| BAT-26-F | SEQ ID No.21 | GACTTCAGCCAGTATATGAAATTGGATATTG | |
| BAT-26-R | SEQ ID No.22 | GTATATGTCAATGAAAACATTTTTTAACCATTCAAC | |
| BAT-26-VIC | SEQ ID No.23 | AGCAGTCAGAGCCCTTAACCTTT | 5'-VIC, 3'-MGB |
| BAT-26-FAM | SEQ ID No.24 | AGGTAAAAAAAAAAAAAAAAAAAAAAAAAAAGG | 5'-FAM, 3'-MGB |

The qPCR reaction solution used in this embodiment includes as follows.

A first qPCR reaction solution for detecting microsatellite loci NR-21; the first qPCR reaction solution has the forward primer shown in SEQ ID No: 1, the reverse primer shown in SEQ ID No: 2, the reference probe shown in SEQ ID No: 3, and the detection probe shown in SEQ ID No: 4; wherein, the nucleotide sequence of SEQ ID No: 3 has a VIC label at its 5' end, and a MGB label at its 3' end; and furthermore, the nucleotide sequence of SEQ ID No: 4 has a FAM label at its 5' end, and has a MGB label at 3' end.

A second qPCR reaction solution for detecting microsatellite loci NR-24; the second qPCR reaction solution has the forward primer shown in SEQ ID No: 5, the reverse primer shown in SEQ ID No: 6, the reference probe shown in SEQ ID No: 7, and the detection probe shown in SEQ ID No: 8; wherein, the nucleotide sequence of SEQ ID No: 7 has a VIC label at its 5' end and a MGB label at its 3' end; and furthermore, the nucleotide sequence of SEQ ID No: 8 has a FAM label at its 5' end, and a MGB label at its 3' end.

A third qPCR reaction solution for detecting microsatellite loci NR-27; the third qPCR reaction solution has the forward primer shown in SEQ ID No: 9, the reverse primer shown in SEQ ID No: 10, the reference probe shown in SEQ ID No: 11, and the detection probe shown in SEQ ID No: 12; wherein, the nucleotide sequence of SEQ ID No: 11 has a VIC label at its 5' end and a MGB label at its 3' end; and furthermore, the nucleotide sequence of SEQ ID No: 12 has a FAM label at its 5' end, and a MGB label at its 3' end.

A fourth qPCR reaction solution for detecting microsatellite loci MONO-27; the fourth qPCR reaction solution has the forward primer shown in SEQ ID No: 13, the reverse primer shown in SEQ ID No: 14, the reference probe shown in SEQ ID No: 15, and the detection probe shown in SEQ ID No: 16; wherein, the nucleotide sequence of SEQ ID No: 15 has a VIC label at its 5' end and a MGB label at its 3' end; and furthermore, the nucleotide sequence of SEQ ID No: 16 has a FAM label at its 5' end, and a MGB label at its 3' end.

A fifth qPCR reaction solution for detecting microsatellite loci BAT-25; the fifth qPCR reaction solution has the forward primer shown in SEQ ID No: 17, the reverse primer shown in SEQ ID No: 18, the reference probe shown in SEQ ID No: 19, and the detection probe shown in SEQ ID No: 20; wherein, the nucleotide sequence of SEQ ID No: 19 has a VIC label at its 5' end and a MGB label at its 3' end; and furthermore, the nucleotide sequence of SEQ ID No: 120 has a FAM label at its 5' end, and a MGB label at its 3' end.

A sixth qPCR reaction solution for detecting microsatellite loci BAT-26; the sixth qPCR reaction solution has the forward primer shown in SEQ ID No: 21, the reverse primer shown in SEQ ID No: 22, the reference probe shown in SEQ ID No: 23, and the detection probe shown in SEQ ID No: 24; wherein, the nucleotide sequence of SEQ ID No: 23 has a VIC label at its 5' end and a MGB label at its 3' end; and furthermore, the nucleotide sequence of SEQ ID No: 24 has a FAM label at its 5' end, and a MGB label at its 3' end.

The components of MSI-qPCR kit in this embodiment are shown as Table 2 bellow.

Table 2 Components of MSI-qPCR kit

| 1 | Negative control | DNA Solution of Cell Line SW480 Genomic | 100 μl | 1 tube |
|---|---|---|---|---|
| 2 | First qPCR reaction solution | NR-21 probe and primer mixture | 100 μl | 1 tube |
| 3 | Second qPCR reaction solution | NR-24 probe and primer mixture | 100μl | 1 tube |
| 4 | Third qPCR reaction solution | NR-27 probe and primer mixture | 100 μl | 1 tube |
| 5 | Fourth qPCR reaction solution | MONO-27 probe and primer mixture | 100 μl | 1 tube |
| 6 | Fifth qPCR reaction solution | BAT-25 probe and primer mixture | 100 μl | 1 tube |
| 7 | Sixth qPCR reaction solution | BAT-26 probe and primer mixture | 100 μl | 1 tube |
| 8 | qPCR premix | PCR MIX | 1 ml | 1 tube |
| 9 | Sterile enzyme-free water | | 1 ml | 1 tube |

(3) MSI-qPCR Reaction

Preparing 12 reaction systems (6 samples with detection sites and 6 negative controls) in eight-strip tubes according to the contents listed in Table 3, with 3 parallels for each sample. After the test samples are mixed and prepared according to the specification and the lid is close, putting them into the qPCR instrument for the following reaction program:

Stage 1: predenaturation 1 cycles: 95° C. for 30 seconds.
Stage 2: qPCR reaction 40 cycles: 95° C. for 5 seconds, 53° C. for 34 seconds.

Where, the heating rate is 4° C./s.

Table 3 MSI-qPCR Reaction System

| Components | Volume |
|---|---|
| Premix Ex Taq™ (Probe qPCR) (2x) | 10.0 μl |
| Primer and Probe Mixtures at Mutation site | 1.6 μl |
| Negative control/Sample | 2.0 μl |
| Sterile water | 6.4 μl |

Be noted, the primer and probe mixtures at mutation site (*i.e.,* qPCR reaction solutions) are primer and probe mixtures of NR-21, NR-24, NR-27, MONO-27, BAT-25, and BAT-26, respectively; the concentration of any one of the forward primer, reverse primer, reference probe, and detection probe in the primer and probe mixture is 10 μM.

(4) Judgement of the Detection Result

Detecting the FFPE sample to obtain the difference between the ΔCt value of the sample and the ΔCt value of the negative control. Namely:

$$\Delta Ct = |Ct_{FAM} - Ct_{VIC}| \quad ;$$

$$\Delta\Delta Ct = |\Delta Ct_{Sample} - \Delta Ct_{Negative}|.$$

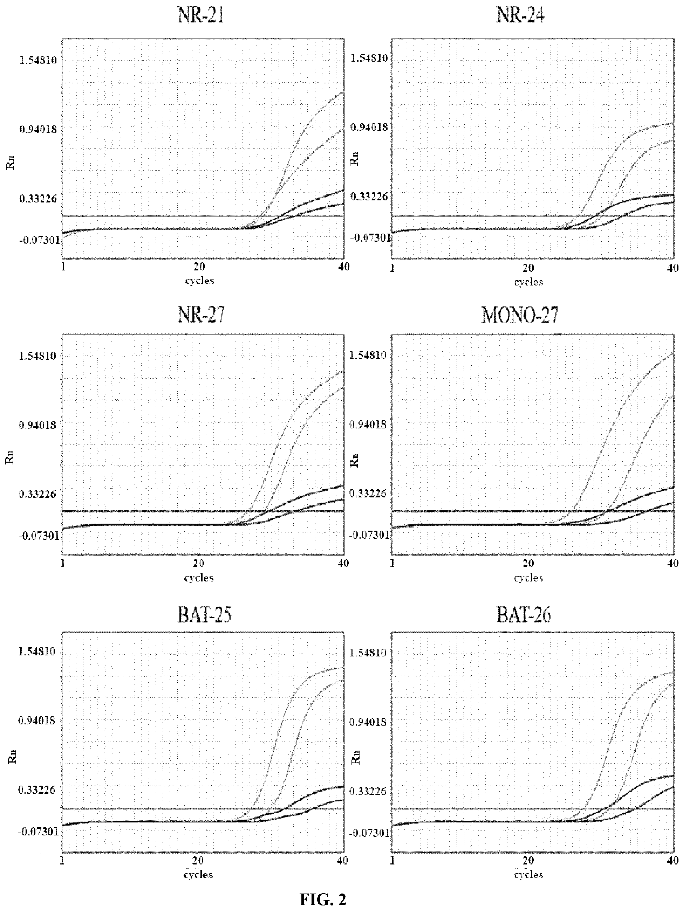

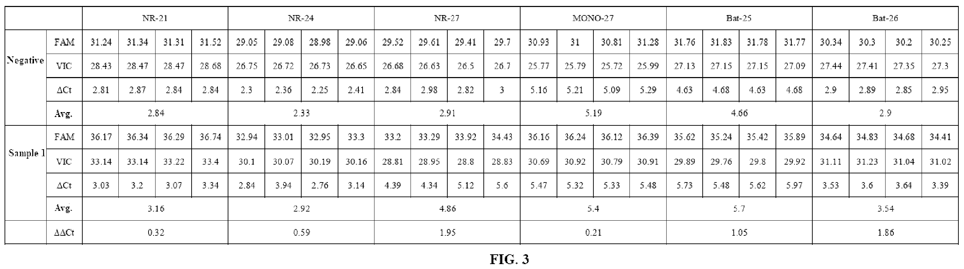

With respect to NR-21, when ΔΔCt > 0.15, the site is determined as positive.
With respect to NR-24, when ΔΔCt > 0.25, the site is determined as positive.
With respect to NR-27, when ΔΔCt > 0.54, the site is determined as positive.
With respect to MONO-27, when ΔΔCt > 0.24, the site is determined as positive.
With respect to BAT-25, when ΔΔCt > 0.23, the site is determined as positive.
With respect to BAT-26, when ΔΔCt > 0.51, the site is determined as positive.
When the number of positive sites in the sample is greater than or equal to 2, the sample with the detection sites is determined to be microsatellite instability MSI, otherwise microsatellite stable MSS.
According to the above judgment criteria, the results obtained by the MSI-qPCR method in this embodiment are shown in FIG. 2 to FIG. 5.
In the drawings, FIG. 2 is a characterization diagram of the qPCR amplification curve of sample 1. FIG. 2 illustrates the Ct values of sample 1 corresponding VIC and FAM fluorescence signals after 1 to 40 cycles in qPCR reaction. FIG. 3 illustrates Ct values, ΔCt values, and ΔΔCt values of VIC, FAM fluorescence signals in sample 1 and its corresponding negative control after qPCR amplification reaction.

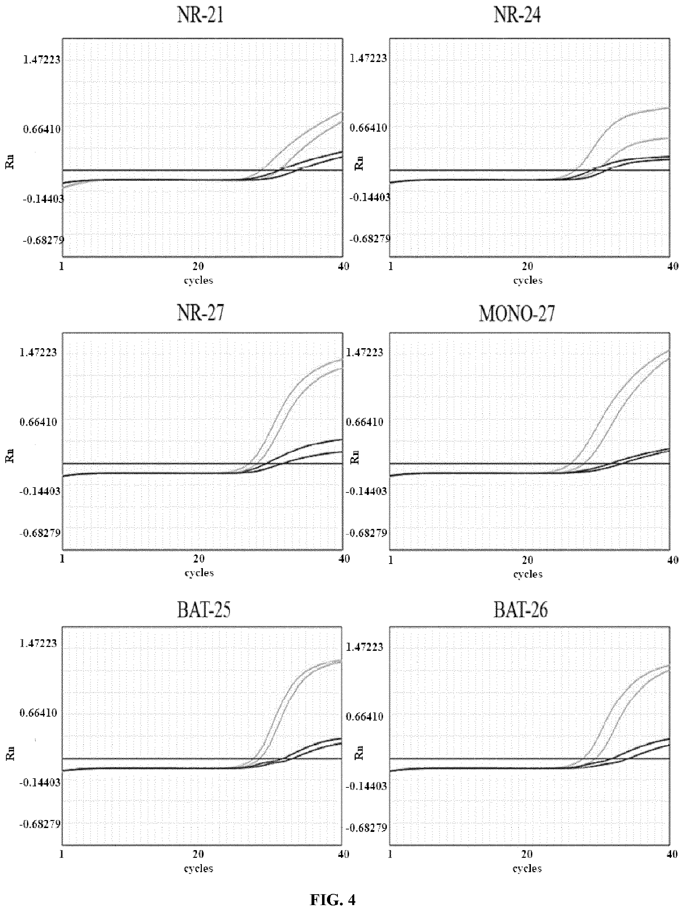

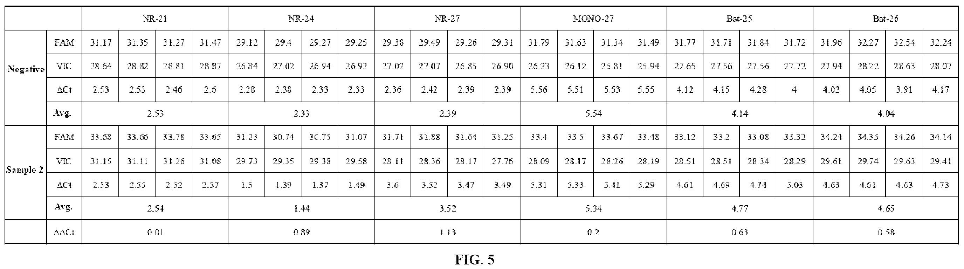

FIG. 4 is a characterization diagram of the qPCR amplification curve of sample 2. FIG. 4 illustrates the Ct values of sample 2 corresponding VIC and FAM fluorescence signals after 1 to 40 cycles in qPCR reaction. FIG. 5 illustrates Ct values, ΔCt values, and ΔΔCt values of VIC, FAM fluorescence signals in sample 2 and its corresponding negative control after qPCR amplification reaction.

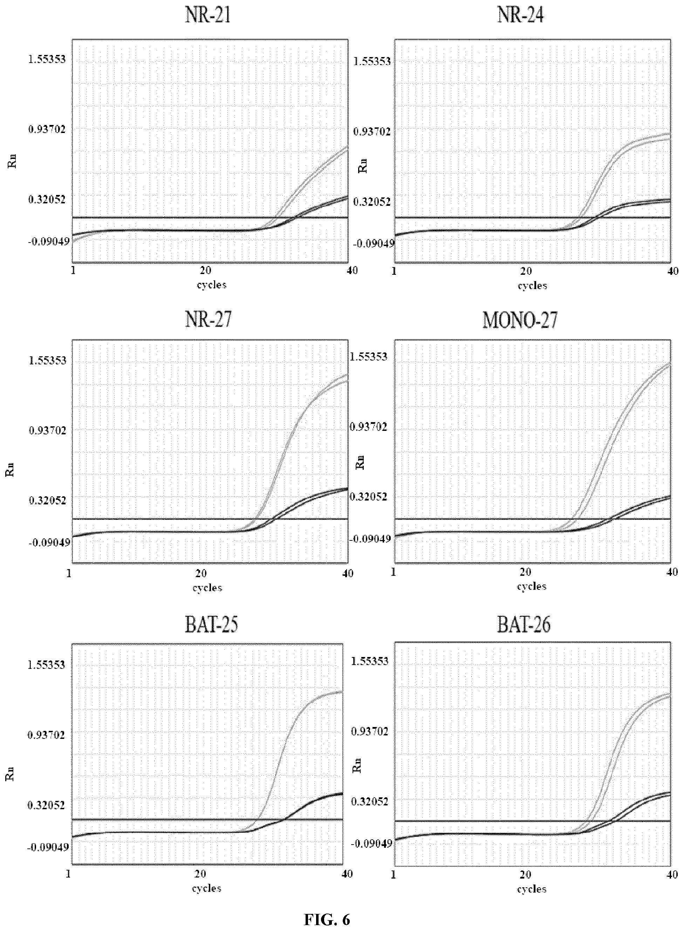

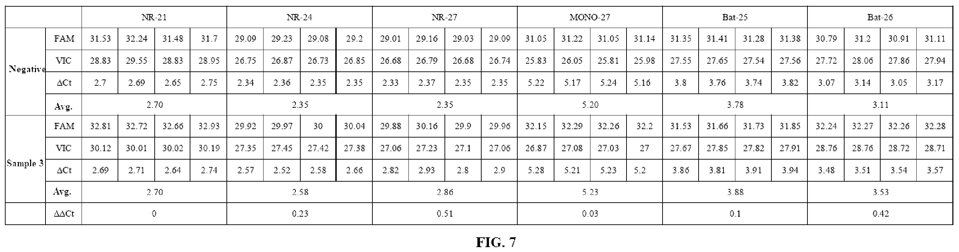

FIG. 6 is a characterization diagram of the qPCR amplification curve of sample 3. FIG. 6 illustrates the Ct values of sample 3 corresponding VIC and FAM fluorescence signals after 1 to 40 cycles in qPCR reaction. FIG. 7 illustrates Ct values, ΔCt values, and ΔΔCt values of VIC, FAM fluorescence signals in sample 3 and its corresponding negative control after qPCR amplification reaction.

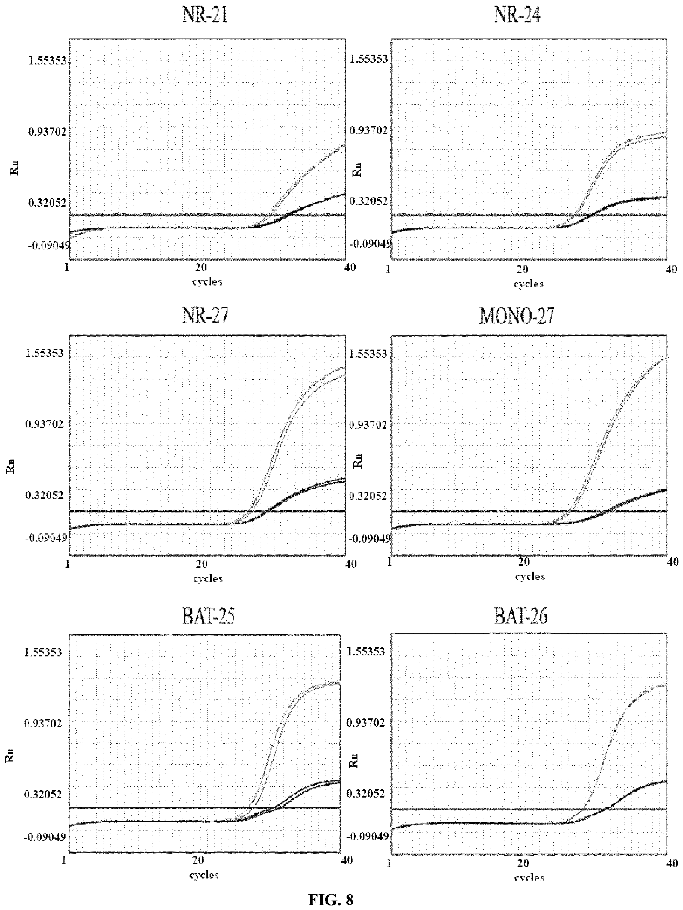

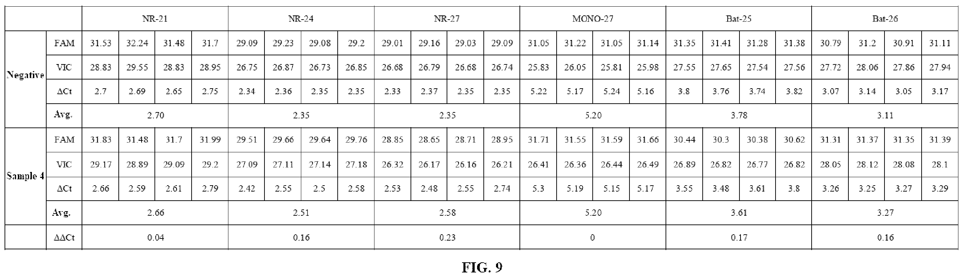

FIG. 8 is a characterization diagram of the qPCR amplification curve of sample 4. FIG. 8 illustrates the Ct values of sample 4 corresponding VIC and FAM fluorescence signals after 1 to 40 cycles in qPCR reaction. FIG. 9 illustrates Ct values, ΔCt values, and ΔΔCt values of VIC, FAM fluorescence signals in sample 4 and its corresponding negative control after qPCR amplification reaction.
According to the results of corresponding sample set in FIG. 3, FIG. 5, FIG. 7, AND FIG. 9, the following results can be obtained.
Five positive sites of sample 1 were detected (NR-21, NR-24, NR-27, BAT-25 and BAT-26), and sample 1 was determined to be MSI, which was consistent with the immunohistochemical (IHC) detection results.
Four positive sites of sample 2 were detected (NR-21, NR-24, NR-27, BAT-25 and BAT-26), and sample 2 was determined to be MSI, which was consistent with the IHC detection results.

Zero positive site of sample 3 was detected, and sample 3 was determined to be MSS, which was consistent with the IHC detection results.

Zero positive site of sample 4 was detected, and sample 4 was determined to be MSS, which was consistent with the IHC detection results.

Based on the above results, it can be concluded that the detection results of the MSI-qPCR method in this embodiment are consistent with the detection results of IHC, while the MSI-qPCR method in this embodiment exhibits faster detection speed, and high sensitivity.

The above descriptions are only some embodiments of the present invention, and are not intended to limit the protection scope of the present invention. Any equivalent device or equivalent process transformation made by using the contents of the description and drawings of the present invention, or directly or indirectly applied to other related technical fields, are similarly included in the scope of patent protection of the present invention

**Claims**

1. A kit for detecting microsatellite instability, **characterized in that** the kit comprises a negative control, a plurality of qPCR reaction solutions, a qPCR premixes and a sterile enzyme-free water;
   the plurality of qPCR reaction solutions are applied respectively one on one to detect microsatellite loci NR-21, NR-24, NR-27, MONO-27, BAT-25, and BAT-26.

2. The kit for detecting microsatellite instability according to claim 1, **characterized in that** the plurality of qPCR reaction solutions comprises:

   a first qPCR reaction solution for detecting microsatellite loci NR-21; the first qPCR reaction solution has the forward primer shown in SEQ ID No: 1, the reverse primer shown in SEQ ID No: 2, the reference probe shown in SEQ ID No: 3, and the detection probe shown in SEQ ID No: 4; wherein, the nucleotide sequence of SEQ ID No: 3 has a VIC label at its 5' end, and a MGB label at its 3' end; and furthermore, the nucleotide sequence of SEQ ID No: 4 has a FAM label at its 5' end, and has a MGB label at 3' end;
   a second qPCR reaction solution for detecting microsatellite loci NR-24; the second qPCR reaction solution has the forward primer shown in SEQ ID No: 5, the reverse primer shown in SEQ ID No: 6, the reference probe shown in SEQ ID No: 7, and the detection probe shown in SEQ ID No: 8; wherein, the nucleotide sequence of SEQ ID No: 7 has a VIC label at its 5' end and a MGB label at its 3' end; and furthermore, the nucleotide sequence of SEQ ID No: 8 has a FAM label at its 5' end, and a MGB label at its 3' end;
   a third qPCR reaction solution for detecting microsatellite loci NR-27; the third qPCR reaction solution has the forward primer shown in SEQ ID No: 9, the reverse primer shown in SEQ ID No: 10, the reference probe shown in SEQ ID No: 11, and the detection probe shown in SEQ ID No: 12; wherein, the nucleotide sequence of SEQ ID No: 11 has a VIC label at its 5' end and a MGB label at its 3' end; and furthermore, the nucleotide sequence of SEQ ID No: 12 has a FAM label at its 5' end, and a MGB label at its 3' end;
   a fourth qPCR reaction solution for detecting microsatellite loci MONO-27; the fourth qPCR reaction solution has the forward primer shown in SEQ ID No: 13, the reverse primer shown in SEQ ID No: 14, the reference probe shown in SEQ ID No: 15, and the detection probe shown in SEQ ID No: 16; wherein, the nucleotide sequence of SEQ ID No: 15 has a VIC label at its 5' end and a MGB label at its 3' end; and furthermore, the nucleotide sequence of SEQ ID No: 16 has a FAM label at its 5' end, and a MGB label at its 3' end;
   a fifth qPCR reaction solution for detecting microsatellite loci BAT-25; the fifth qPCR reaction solution has the forward primer shown in SEQ ID No: 17, the reverse primer shown in SEQ ID No: 18, the reference probe shown in SEQ ID No: 19, and the detection probe shown in SEQ ID No: 20; wherein, the nucleotide sequence of SEQ ID No: 19 has a VIC label at its 5' end and a MGB label at its 3' end; and furthermore, the nucleotide sequence of SEQ ID No: 120 has a FAM label at its 5' end, and a MGB label at its 3' end;
   a sixth qPCR reaction solution for detecting microsatellite loci BAT-26; the sixth qPCR reaction solution has the forward primer shown in SEQ ID No: 21, the reverse primer shown in SEQ ID No: 22, the reference probe shown in SEQ ID No: 23, and the detection probe shown in SEQ ID No: 24; wherein, the nucleotide sequence of SEQ ID No: 23 has a VIC label at its 5' end and a MGB label at its 3' end; and furthermore, the nucleotide sequence of SEQ ID No: 24 has a FAM label at its 5' end, and a MGB label at its 3' end.

3. The kit for detecting microsatellite instability according to claim 2, **characterized in that** the negative control is a genomic DNA solution of cell line SW480 at a concentration of 10 ng/$\mu$l;

   the qPCR premixes is Premix Ex Taq, which includes Taq HS, dNTP Mixture, $Mg^{2+}$ and Tli RNaseH;

the concentrations of the forward primer, the reverse primer, the reference probe, and the detection probe in the plurality of qPCR reaction solutions are 10 μM.

4. A non-therapeutic method for detecting microsatellite instability, **characterized in that** the non-therapeutic method comprises the kit for detecting microsatellite instability according to any of claims 1-3, the non-therapeutic method comprises the following steps:

S01, taking all components of the kit, mixing the components according to preset conditions, and preparing a plurality of qPCR reaction system mixtures including samples with detection sites, and negative controls;

502, performing qPCR amplification reactions according to preset conditions, and after the reaction, characterizing the plurality of qPCR reaction system mixtures to obtain Ct values having VIC and FAM fluorescence signals; calculating the difference ΔCt for the Ct values of the FAM and VIC signals at the same detection site of the sample;

503, calculating the difference between the ΔCt value of the sample detection site and the ΔCt value of the negative control at the same site as the sample detection site; namely,

$$\Delta Ct = |Ct_{FAM} - Ct_{VIC}| \quad ;$$

$$\Delta\Delta Ct = \left|\Delta Ct_{Sample} - \Delta Ct_{Negative}\right| \quad ;$$

S04, obtaining ΔΔCt and judging it according to preset conditions, and outputting the sample detection result as microsatellite instability MSI or microsatellite stability MSS according to the judgment result.

5. The non-therapeutic method for detecting microsatellite instability according to claim 4, **characterized in that** the plurality of qPCR reaction system mixtures are 12 qPCR reaction system mixtures, including 6 samples with detection sites and 6 negative controls, corresponding to the microsatellite loci NR-21, NR-24, NR-27, MONO-27, BAT-25, BAT-26, respectively.

6. The non-therapeutic method for detecting microsatellite instability according to claim 4, **characterized in that** the step S01 includes the following steps:
taking all components of the kit, melting the components on ice, shaking and mixing them evenly, and then briefly centrifuging for a few seconds to prepare the plurality of qPCR reaction system mixtures including samples with detection sites and negative controls.

7. The non-therapeutic method for detecting microsatellite instability according to claim 4, **characterized in that** in the step S02, the conditions for the qPCR amplification action is as follows:

predenaturation 95° C. for 30 seconds
qPCR reaction 95° C. for 5 seconds, 53 degree annealing extension for 34 seconds, 40 cycles.

8. The non-therapeutic method for detecting microsatellite instability according to claim 4, **characterized in that** in the step S04,

with respect to NR-21, when ΔΔCt > 0.15, the site is determined as positive;
with respect to NR-24, when ΔΔCt > 0.25, the site is determined as positive;
with respect to NR-27, when ΔΔCt > 0.54, the site is determined as positive;
with respect to MONO-27, when ΔΔCt > 0.24, the site is determined as positive;
with respect to BAT-25, when ΔΔCt > 0.23, the site is determined as positive;
with respect to BAT-26, when ΔΔCt > 0.51, the site is determined as positive;
when the number of positive sites in the sample is greater than or equal to 2, the sample with the detection sites is determined to be microsatellite instability MSI, otherwise microsatellite stable MSS.

9. A non-therapeutic method for detecting microsatellite instability in tissues or ctDNA, **characterized in that** the non-therapeutic method comprises the non-therapeutic method for detecting microsatellite instability according to any one of claims 4-8.

**10.** A non-therapeutic kit for detecting microsatellite instability in tissues or ctDNA, **characterized in that** the non-therapeutic kit comprises the kit for detecting microsatellite instability according to any one of claims 1-3.

WT **Allele** (VIC⁺FAM⁺)

MSI **Allele** (VIC⁺FAM⁻ᐟˡᵒʷ)

Fig. 1

**FIG. 2**

|  |  | NR-21 |  |  |  | NR-24 |  |  |  | NR-27 |  |  |  | MONO-27 |  |  |  | Bat-25 |  |  |  | Bat-26 |  |  |  |
|---|---|---|---|---|---|---|---|---|---|---|---|---|---|---|---|---|---|---|---|---|---|---|---|---|---|
| Negative | FAM | 31.24 | 31.34 | 31.31 | 31.52 | 29.05 | 29.08 | 28.98 | 29.06 | 29.52 | 29.61 | 29.41 | 29.7 | 30.93 | 31 | 30.81 | 31.28 | 31.76 | 31.83 | 31.78 | 31.77 | 30.34 | 30.3 | 30.2 | 30.25 |
|  | VIC | 28.43 | 28.47 | 28.47 | 28.68 | 26.75 | 26.72 | 26.73 | 26.65 | 26.68 | 26.63 | 26.5 | 26.7 | 25.77 | 25.79 | 25.72 | 25.99 | 27.13 | 27.15 | 27.15 | 27.09 | 27.44 | 27.41 | 27.35 | 27.3 |
|  | ΔCt | 2.81 | 2.87 | 2.84 | 2.84 | 2.3 | 2.36 | 2.25 | 2.41 | 2.84 | 2.98 | 2.82 | 3 | 5.16 | 5.21 | 5.09 | 5.29 | 4.63 | 4.68 | 4.63 | 4.68 | 2.9 | 2.89 | 2.85 | 2.95 |
|  | Avg. | 2.84 |  |  |  | 2.33 |  |  |  | 2.91 |  |  |  | 5.19 |  |  |  | 4.66 |  |  |  | 2.9 |  |  |  |
| Sample 1 | FAM | 36.17 | 36.34 | 36.29 | 36.74 | 32.94 | 33.01 | 32.95 | 33.3 | 33.2 | 33.29 | 33.92 | 34.43 | 36.16 | 36.24 | 36.12 | 36.39 | 35.62 | 35.24 | 35.42 | 35.89 | 34.64 | 34.83 | 34.68 | 34.41 |
|  | VIC | 33.14 | 33.14 | 33.22 | 33.4 | 30.1 | 30.07 | 30.19 | 30.16 | 28.81 | 28.95 | 28.8 | 28.83 | 30.69 | 30.92 | 30.79 | 30.91 | 29.89 | 29.76 | 29.8 | 29.92 | 31.11 | 31.23 | 31.04 | 31.02 |
|  | ΔCt | 3.03 | 3.2 | 3.07 | 3.34 | 2.84 | 3.94 | 2.76 | 3.14 | 4.39 | 4.34 | 5.12 | 5.6 | 5.47 | 5.32 | 5.33 | 5.48 | 5.73 | 5.48 | 5.62 | 5.97 | 3.53 | 3.6 | 3.64 | 3.39 |
|  | Avg. | 3.16 |  |  |  | 2.92 |  |  |  | 4.86 |  |  |  | 5.4 |  |  |  | 5.7 |  |  |  | 3.54 |  |  |  |
|  | ΔΔCt | 0.32 |  |  |  | 0.59 |  |  |  | 1.95 |  |  |  | 0.21 |  |  |  | 1.05 |  |  |  | 1.86 |  |  |  |

**FIG. 3**

FIG. 4

| | | NR-21 | | | | NR-24 | | | | NR-27 | | | | MONO-27 | | | | Bat-25 | | | | Bat-26 | | | |
|---|---|---|---|---|---|---|---|---|---|---|---|---|---|---|---|---|---|---|---|---|---|---|---|---|---|
| Negative | FAM | 31.17 | 31.35 | 31.27 | 31.47 | 29.12 | 29.4 | 29.27 | 29.25 | 29.38 | 29.49 | 29.26 | 29.31 | 31.79 | 31.63 | 31.34 | 31.49 | 31.77 | 31.71 | 31.84 | 31.72 | 31.96 | 32.27 | 32.54 | 32.24 |
| | VIC | 28.64 | 28.82 | 28.81 | 28.87 | 26.84 | 27.02 | 26.94 | 26.92 | 27.02 | 27.07 | 26.85 | 26.90 | 26.23 | 26.12 | 25.81 | 25.94 | 27.65 | 27.56 | 27.56 | 27.72 | 27.94 | 28.22 | 28.63 | 28.07 |
| | ΔCt | 2.53 | 2.53 | 2.46 | 2.6 | 2.28 | 2.38 | 2.33 | 2.33 | 2.36 | 2.42 | 2.39 | 2.39 | 5.56 | 5.51 | 5.53 | 5.55 | 4.12 | 4.15 | 4.28 | 4 | 4.02 | 4.05 | 3.91 | 4.17 |
| | Avg. | 2.53 | | | | 2.33 | | | | 2.39 | | | | 5.54 | | | | 4.14 | | | | 4.04 | | | |
| Sample 2 | FAM | 33.68 | 33.66 | 33.78 | 33.65 | 31.23 | 30.74 | 30.75 | 31.07 | 31.71 | 31.88 | 31.64 | 31.25 | 33.4 | 33.5 | 33.67 | 33.48 | 33.12 | 33.2 | 33.08 | 33.32 | 34.24 | 34.35 | 34.26 | 34.14 |
| | VIC | 31.15 | 31.11 | 31.26 | 31.08 | 29.73 | 29.35 | 29.38 | 29.58 | 28.11 | 28.36 | 28.17 | 27.76 | 28.09 | 28.17 | 28.26 | 28.19 | 28.51 | 28.51 | 28.34 | 28.29 | 29.61 | 29.74 | 29.63 | 29.41 |
| | ΔCt | 2.53 | 2.55 | 2.52 | 2.57 | 1.5 | 1.39 | 1.37 | 1.49 | 3.6 | 3.52 | 3.47 | 3.49 | 5.31 | 5.33 | 5.41 | 5.29 | 4.61 | 4.69 | 4.74 | 5.03 | 4.63 | 4.61 | 4.63 | 4.73 |
| | Avg. | 2.54 | | | | 1.44 | | | | 3.52 | | | | 5.34 | | | | 4.77 | | | | 4.65 | | | |
| | ΔΔCt | 0.01 | | | | 0.89 | | | | 1.13 | | | | 0.2 | | | | 0.63 | | | | 0.58 | | | |

**FIG. 5**

FIG. 6

| | | NR-21 | | | | NR-24 | | | | NR-27 | | | | MONO-27 | | | | Bat-25 | | | | Bat-26 | | | |
|---|---|---|---|---|---|---|---|---|---|---|---|---|---|---|---|---|---|---|---|---|---|---|---|---|---|
| Negative | FAM | 31.53 | 32.24 | 31.48 | 31.7 | 29.09 | 29.23 | 29.08 | 29.2 | 29.01 | 29.16 | 29.03 | 29.09 | 31.05 | 31.22 | 31.05 | 31.14 | 31.35 | 31.41 | 31.28 | 31.38 | 30.79 | 31.2 | 30.91 | 31.11 |
| | VIC | 28.83 | 29.55 | 28.83 | 28.95 | 26.75 | 26.87 | 26.73 | 26.85 | 26.68 | 26.79 | 26.68 | 26.74 | 25.83 | 26.05 | 25.81 | 25.98 | 27.55 | 27.65 | 27.54 | 27.56 | 27.72 | 28.06 | 27.86 | 27.94 |
| | $\Delta$Ct | 2.7 | 2.69 | 2.65 | 2.75 | 2.34 | 2.36 | 2.35 | 2.35 | 2.33 | 2.37 | 2.35 | 2.35 | 5.22 | 5.17 | 5.24 | 5.16 | 3.8 | 3.76 | 3.74 | 3.82 | 3.07 | 3.14 | 3.05 | 3.17 |
| | Avg. | 2.70 | | | | 2.35 | | | | 2.35 | | | | 5.20 | | | | 3.78 | | | | 3.11 | | | |
| Sample 3 | FAM | 32.81 | 32.72 | 32.66 | 32.93 | 29.92 | 29.97 | 30 | 30.04 | 29.88 | 30.16 | 29.9 | 29.96 | 32.15 | 32.29 | 32.26 | 32.2 | 31.53 | 31.66 | 31.73 | 31.85 | 32.24 | 32.27 | 32.26 | 32.28 |
| | VIC | 30.12 | 30.01 | 30.02 | 30.19 | 27.35 | 27.45 | 27.42 | 27.38 | 27.06 | 27.23 | 27.1 | 27.06 | 26.87 | 27.08 | 27.03 | 27 | 27.67 | 27.85 | 27.82 | 27.91 | 28.76 | 28.76 | 28.72 | 28.71 |
| | $\Delta$Ct | 2.69 | 2.71 | 2.64 | 2.74 | 2.57 | 2.52 | 2.58 | 2.66 | 2.82 | 2.93 | 2.8 | 2.9 | 5.28 | 5.21 | 5.23 | 5.2 | 3.86 | 3.81 | 3.91 | 3.94 | 3.48 | 3.51 | 3.54 | 3.57 |
| | Avg. | 2.70 | | | | 2.58 | | | | 2.86 | | | | 5.23 | | | | 3.88 | | | | 3.53 | | | |
| | $\Delta\Delta$Ct | 0 | | | | 0.23 | | | | 0.51 | | | | 0.03 | | | | 0.1 | | | | 0.42 | | | |

**FIG. 7**

FIG. 8

| | | NR-21 | | | | NR-24 | | | | NR-27 | | | | MONO-27 | | | | Bat-25 | | | | Bat-26 | | | |
|---|---|---|---|---|---|---|---|---|---|---|---|---|---|---|---|---|---|---|---|---|---|---|---|---|---|
| Negative | FAM | 31.53 | 32.24 | 31.48 | 31.7 | 29.09 | 29.23 | 29.08 | 29.2 | 29.01 | 29.16 | 29.03 | 29.09 | 31.05 | 31.22 | 31.05 | 31.14 | 31.35 | 31.41 | 31.28 | 31.38 | 30.79 | 31.2 | 30.91 | 31.11 |
| | VIC | 28.83 | 29.55 | 28.83 | 28.95 | 26.75 | 26.87 | 26.73 | 26.85 | 26.68 | 26.79 | 26.68 | 26.74 | 25.83 | 26.05 | 25.81 | 25.98 | 27.55 | 27.65 | 27.54 | 27.56 | 27.72 | 28.06 | 27.86 | 27.94 |
| | ΔCt | 2.7 | 2.69 | 2.65 | 2.75 | 2.34 | 2.36 | 2.35 | 2.35 | 2.33 | 2.37 | 2.35 | 2.35 | 5.22 | 5.17 | 5.24 | 5.16 | 3.8 | 3.76 | 3.74 | 3.82 | 3.07 | 3.14 | 3.05 | 3.17 |
| | Avg. | 2.70 | | | | 2.35 | | | | 2.35 | | | | 5.20 | | | | 3.78 | | | | 3.11 | | | |
| Sample 4 | FAM | 31.83 | 31.48 | 31.7 | 31.99 | 29.51 | 29.66 | 29.64 | 29.76 | 28.85 | 28.65 | 28.71 | 28.95 | 31.71 | 31.55 | 31.59 | 31.66 | 30.44 | 30.3 | 30.38 | 30.62 | 31.31 | 31.37 | 31.35 | 31.39 |
| | VIC | 29.17 | 28.89 | 29.09 | 29.2 | 27.09 | 27.11 | 27.14 | 27.18 | 26.32 | 26.17 | 26.16 | 26.21 | 26.41 | 26.36 | 26.44 | 26.49 | 26.89 | 26.82 | 26.77 | 26.82 | 28.05 | 28.12 | 28.08 | 28.1 |
| | ΔCt | 2.66 | 2.59 | 2.61 | 2.79 | 2.42 | 2.55 | 2.5 | 2.58 | 2.53 | 2.48 | 2.55 | 2.74 | 5.3 | 5.19 | 5.15 | 5.17 | 3.55 | 3.48 | 3.61 | 3.8 | 3.26 | 3.25 | 3.27 | 3.29 |
| | Avg. | 2.66 | | | | 2.51 | | | | 2.58 | | | | 5.20 | | | | 3.61 | | | | 3.27 | | | |
| | ΔΔCt | 0.04 | | | | 0.16 | | | | 0.23 | | | | 0 | | | | 0.17 | | | | 0.16 | | | |

**FIG. 9**

Europäisches
Patentamt

European
Patent Office

Office européen
des brevets

# EUROPEAN SEARCH REPORT

Application Number

EP 22 21 7192

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 109 097 478 A (ZHEJIANG SHUWEN BIOTECH CO LTD) 28 December 2018 (2018-12-28) * see whole doc. esp. claims * | 1-10 | INV. C12Q1/6886 C12Q1/6851 |
| X | WO 2019/162240 A1 (HOFFMANN LA ROCHE [CH]; ROCHE DIAGNOSTICS GMBH [DE] ET AL.) 29 August 2019 (2019-08-29) * see whole doc., esp. claims and page 17, l. 1 ff * | 1-10 | |
| X | CN 109 337 985 A (GUANGZHOU FULENGEN CO LTD) 15 February 2019 (2019-02-15) * see whole doc., esp. claims * | 1-10 | |
| X | CN 114 107 453 A (IPRABYE BIOTECHNOLOGY SUZHOU CO LTD) 1 March 2022 (2022-03-01) * see whole doc. esp. claims * | 1-10 | |
| A | WO 2016/112351 A1 (BIO RAD LABORATORIES [US]; DAVID GLADSTONE INST J [US]) 14 July 2016 (2016-07-14) * see whole doc. esp. claims and Figures * | 1-10 | TECHNICAL FIELDS SEARCHED (IPC) C12Q |

The present search report has been drawn up for all claims

| Place of search | Date of completion of the search | Examiner |
|---|---|---|
| Munich | 10 August 2023 | Mueller, Frank |

EPO FORM 1503 03.82 (P04C01)

## ANNEX TO THE EUROPEAN SEARCH REPORT
## ON EUROPEAN PATENT APPLICATION NO.

EP 22 21 7192

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2023

| Patent document cited in search report | | Publication date | Patent family member(s) | | Publication date |
|---|---|---|---|---|---|
| CN 109097478 | A | 28-12-2018 | NONE | | |
| WO 2019162240 | A1 | 29-08-2019 | CN | 111670254 A | 15-09-2020 |
| | | | EP | 3755813 A1 | 30-12-2020 |
| | | | JP | 2021513858 A | 03-06-2021 |
| | | | US | 2019256925 A1 | 22-08-2019 |
| | | | WO | 2019162240 A1 | 29-08-2019 |
| CN 109337985 | A | 15-02-2019 | NONE | | |
| CN 114107453 | A | 01-03-2022 | NONE | | |
| WO 2016112351 | A1 | 14-07-2016 | CN | 107406842 A | 28-11-2017 |
| | | | CN | 113388670 A | 14-09-2021 |
| | | | EP | 3242938 A1 | 15-11-2017 |
| | | | US | 2016208319 A1 | 21-07-2016 |
| | | | US | 2019203274 A1 | 04-07-2019 |
| | | | US | 2022112544 A1 | 14-04-2022 |
| | | | WO | 2016112351 A1 | 14-07-2016 |

EPO FORM P0459

For more details about this annex : see Official Journal of the European Patent Office, No. 12/82